# FASCICULE DE BREVET EUROPEEN

(11) **EP 1 560 620 B1**
(45) Date de publication et mention de la délivrance du brevet: **25.07.2007**
(21) Numéro de dépôt: 03778450.1
(22) Date de dépôt: 24.10.2003
(51) Int. Cl.: A61M 15/00

(54) **DISPOSITIF DE DISTRIBUTION DE PRODUIT FLUIDE AVEC INDICATEUR DE DOSE**
FLUSSIGKEITSSPENDER MIT ZÄHLEINRICHTUNG DER DOSISEINHEITEN
FLUID PRODUCT DISPENSING DEVICE WITH DOSE INDICATOR

(30) Priorité: 28.10.2002 FR 0213472
(43) Date de publication de la demande: 10.08.2005
(73) Titulaire: Valois SAS, 27110 Le Neubourg (FR)
(72) Inventeur: BRUNA, Pascal, F-76300 Sotteville les Rouen (FR)
(74) Mandataire: CAPRI
(86) Numéro de dépôt international: PCT/FR2003/003155
(87) Numéro de publication internationale: WO 2004/039443

(56) Documents cités:
- EP-A- 0 684 047
- WO-A-02/058771
- DE-A- 4 340 593
- US-A- 5 544 647
- US-A- 6 029 659

## Description

La présente invention concerne un dispositif de distribution de produit fluide et plus particulièrement un tel dispositif incorporant un indicateur de dose.

Les indicateurs de dose, et plus particulièrement les compteurs de doses, sont bien connus pour être utilisés avec des dispositifs de distribution de produit fluide. Par produit fluide, on entend les produits gazeux, liquides, pâteux, ou pulvérulents. Ces compteurs sont généralement destinés à afficher le nombre de doses distribuées ou le nombre de doses restant à distribuer, et divers types de compteurs ont été réalisés. Une première famille de compteur est formée par les dispositifs mécaniques comportant généralement des roues de comptage qui sont actionnées en rotation lors de l'actionnement du dispositif. Ces compteurs présentent des inconvénients en ce qu'ils sont généralement assez encombrants et nécessitent une modification structurelle importante du dispositif pour permettre l'adaptation du compteur. Par ailleurs, ces compteurs étant limités dimensionnellement, le nombre de doses pouvant être comptées est forcément limité, et lorsqu'un grand nombre de doses est disposé dans le réservoir, par exemple deux cents, l'affichage devient généralement très petit et donc peu lisible, notamment pour les personnes âgées. Un autre type de compteur est formé par les compteurs électroniques. Ces compteurs comportent un affichage électronique qui est modifié à chaque actionnement du dispositif. Ces compteurs électroniques nécessitent une alimentation électrique et sont généralement également relativement encombrants. Selon le type de source d'énergie utilisée, le risque existe qu'après un temps de stockage relativement important, les sources d'énergies sont épuisées, de sorte que le compteur ne peut plus fonctionner. Ceci peut notamment arriver avec les piles ou accumulateurs. Les documents EP-A-684 047, US-A-6 029 659, WO-A-02/058771 et US-A-5 544 647 divulguent l'utilisation d'afficheurs à cristaux liquides (LCD) qui nécessitent une alimentation d'énergie (généralement au moyen d'une pile) pour fonctionner.

La présente invention a pour but de fournir un dispositif de distribution de produit fluide qui ne reproduit pas les inconvénients susmentionnés.

Plus particulièrement, la présente invention a pour but de fournir un dispositif de distribution de produit fluide comportant un indicateur de dose peu encombrant et adaptable à tout type de dispositif de distribution de produit fluide existant sans modifier sa structure ou ses dimensions externes.

La présente invention a aussi pour but de fournir un tel dispositif permettant de compter de manière lisible un nombre quelconque de doses.

La présente invention a également pour but de fournir un tel dispositif simple et peu coûteux à fabriquer et à assembler, et fonctionnant de manière fiable, tout en nécessitant qu'une énergie minimale pour son fonctionnement.

La présente invention a donc pour objet un dispositif de distribution de produit fluide comportant, un corps incorporant un orifice de distribution, un réservoir contenant le produit fluide, et un organe de distribution, tel qu'une pompe ou une valve doseuse pour distribuer sélectivement le produit contenu dans le réservoir, le dispositif comportant un indicateur de dose comprenant des moyens d'affichage électroniques, lesdits moyens d'affichage comportant un afficheur permanent, tel qu'un afficheur nématique, bistable, ne nécessitant aucune énergie pour maintenir l'affichage inchangé et ne nécessitant qu'une très faible énergie pour modifier ledit affichage.

Avantageusement, l'afficheur est du type à cristaux liquides (LCD).

Avantageusement, l'afficheur comporte des cristaux nématiques bistables.

Selon une première variante de réalisation, l'énergie nécessaire pour modifier l'affichage est fournie par une pile ou accumulateur

Selon une seconde variante de réalisation avantageuse, l'énergie nécessaire pour modifier l'affichage est créée lors de l'actionnement du dispositif.

Avantageusement, l'interaction entre deux parties du dispositif se déplaçant l'une par rapport à l'autre lors de l'actionnement du dispositif est transformée par un convertisseur électromécanique en une impulsion électrique utilisée pour modifier l'affichage.

Avantageusement, ladite interaction est un frottement ou une percussion d'une partie du dispositif sur une autre partie du dispositif lors de l'actionnement.

Avantageusement, le réservoir est déplaçable lors de l'actionnement par rapport au corps du dispositif, ledit corps comportant un contacteur coopérant avec ledit réservoir, l'interaction entre ledit réservoir et ledit contacteur créant l'impulsion électrique nécessaire pour modifier l'affichage.

En variante, un percuteur est déplacé contre un contacteur lors de l'actionnement du dispositif, ledit contacteur étant fixe par rapport audit corps, et ledit percuteur coopérant avec un ressort.

Avantageusement, ledit indicateur de dose indique le nombre de doses de produit distribué ou restant à distribuer du réservoir.

Avantageusement, ledit indicateur de dose a une structure mince, de sorte qu'il est adaptable à un dispositif de distribution de produit fluide sans modifier ses dimensions externes.

D'autres caractéristiques et avantages de la présente invention apparaîtront plus clairement au cours de la description détaillée suivante, faite en référence aux dessins joints, donnés à titre d'exemples non limitatifs et sur lesquels,
la figure 1 est une vue de côté très schématique, montrant un dispositif de distribution de produit fluide selon un mode de réalisation de la présente invention ;
la figure 2 est une vue de face très schématique et en section du dispositif de la figure 1, et
la figure 3 est une vue schématique de côté en section transversale d'une variante de réalisation de l'invention.

L'exemple de réalisation représenté sur les figures est un inhalateur, généralement désigné par le terme MDI (Metered Dose Inhaler). Cet inhalateur comporte un réservoir 10 contenant un produit fluide et un gaz propulseur, une valve doseuse 15 étant assemblée sur le réservoir 10 pour distribuer des doses de produit à travers un embout buccal 5 formé dans un corps 1 qui reçoit le réservoir 10. L'actionnement de ce dispositif est réalisé généralement en déplaçant axialement le réservoir 10 à l'intérieur du corps 1, ce qui actionne la valve pour distribuer une dose de produit. Il est ici précisé que la présente invention ne se limite pas à ce type de dispositif, mais qu'elle est au contraire adaptable à tout dispositif de distribution de produit fluide, et notamment des dispositifs comportant des pompes plutôt que des valves fonctionnant avec un gaz propulseur.

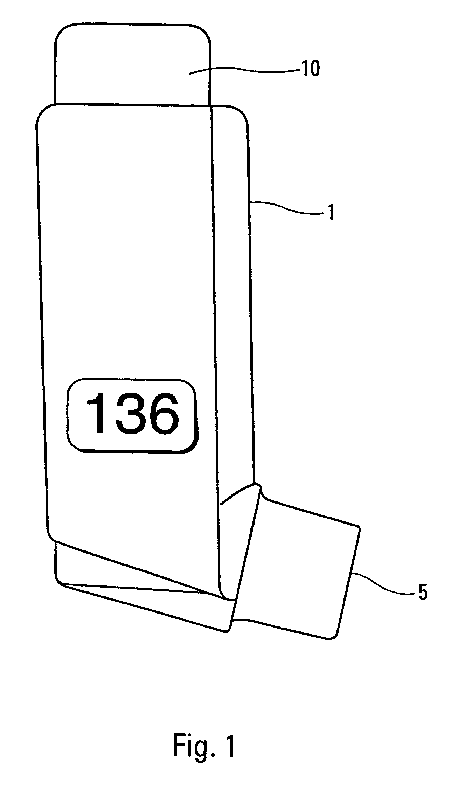

Selon l'invention, le dispositif comporte un indicateur de dose. Cet indicateur est de préférence utilisé pour compter le nombre de doses qui a été distribué, ou qui reste à distribuer à partir du réservoir 10. Cet indicateur comporte des moyens d'affichage électroniques 20, de préférence de grande dimension comme représentée sur la figure 1. Ceci permet à toute personne, même les personnes âgées ayant une vue moins performante, de lire le chiffre affiché par les moyens d'affichage 21, même lorsque le nombre de doses est très important et que le compteur est destiné à compter plusieurs centaines de doses.

Selon l'invention, les moyens d'affichage 20 comportent un afficheur permanent ou rémanent 21. Cet afficheur permanent 21 peut être du type à cristaux liquides (LCD), par exemple, un afficheur à cristaux nématiques bistables. Un afficheur permanent ou rémanent ne nécessite aucune énergie pour maintenir l'affichage inchangé, de sorte qu'entre deux actionnements, aucune source d'énergie n'est nécessaire pour que le chiffre affiché par les moyens d'affichage reste visible pour l'utilisateur. Il se distingue donc des afficheurs à cristaux liquides (LCD) habituels qui ne fonctionnent qu'avec une alimentation en énergie, généralement fournie par une pile. C'est seulement au moment où l'affichage doit être modifié, c'est-à-dire lors d'un actionnement du dispositif, qu'une très faible énergie est nécessaire pour modifier ledit affichage. Cette très faible énergie peut être fournie par une pile ou un accumulateur. De préférence, toutefois, elle peut être créée au moment de l'actionnement du dispositif comme cela sera décrit ci-après, auquel cas aucune pile ou accumulateur n'est nécessaire.

Dans le mode de réalisation préféré, dans lequel l'indicateur fonctionne sans pile ni accumulateur, l'énergie nécessaire pour modifier l'affichage peut être créée par l'interaction entre deux parties du dispositif qui se déplacent l'une par rapport à l'autre lors de l'actionnement. Cette interaction, qui peut par exemple être un frottement ou une percussion d'une partie sur une autre, est ensuite transformée en une ou plusieurs impulsions électriques suffisante(s) pour permettre la modification de l'affichage. Avantageusement, l'interaction peut être créée entre le réservoir 10 et une partie du corps 1, ces deux parties se déplaçant l'une par rapport à l'autre lors de l'actionnement.

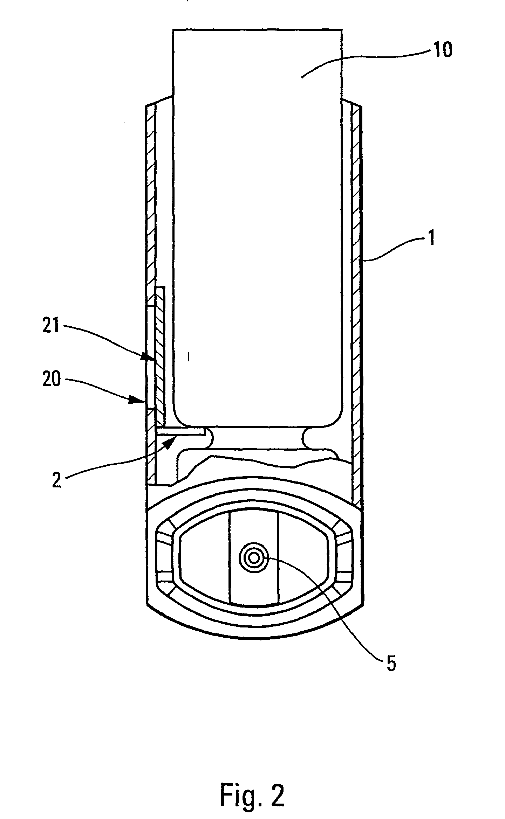

La figure 2 montre un mode de réalisation particulier, dans lequel un contacteur 2 est solidaire du réservoir 10, ledit contacteur venant percuter l'afficheur 21 lorsque le réservoir 10 est ramené vers sa position de repos après distribution de la dose. Bien entendu, le contacteur pourrait être solidaire du corps 1 et coopérer avec le réservoir, ou plus généralement un frottement entre le réservoir et toute pièce fixe solidaire du corps pourrait être suffisante pour créer l'impulsion électrique nécessaire pour modifier l'affichage de l'afficheur. Cette impulsion électrique, typiquement d'une durée de 1 à 50 ms et de l'ordre de 10 000 à 50 000 Volts, est alors traitée par un circuit électronique approprié 25 afin de commander l'afficheur 21.

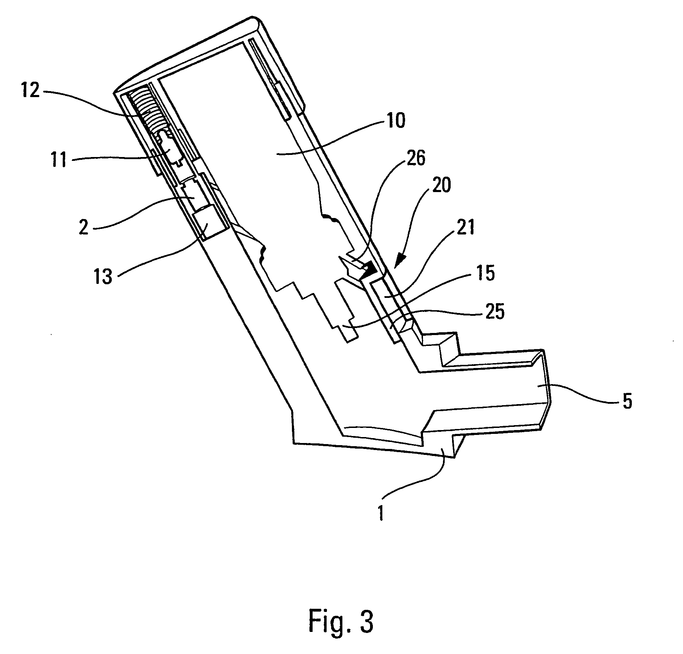

La figure 3 montre une variante de réalisation, dans laquelle un générateur d'impulsions électriques du type pierre à briquet est utilisé. Ce système comporte un percuteur 11 coopérant avec un ressort 12 et sollicité, lors de l'actionnement, contre un contacteur 2. Ce contacteur 2, comprenant avantageusement une céramique solidaire d'une enclume 13, est de préférence fixe par rapport au corps 1. Des fils d'alimentation 26 transmettent l'impulsion au circuit électronique 25, qui la transforme pour commander l'afficheur 21 afin de modifier l'affichage.

Plus généralement, on peut prévoir de convertir l'effort ou le déplacement mécanique lors de l'actionnement du dispositif en signal électrique. On peut utiliser un actionneur piézoélectrique, une bobine électromagnétique ou tout autre dispositif de conversion électromécanique connue par l'homme du métier. Plus particulièrement, un système du type pierre à briquet ou une céramique piézoélectrique, telle que celle utilisée dans les allume-gaz, est utilisable. D'autres variantes sont ainsi envisageables. Par exemple, un générateur thermoélectrique serait utilisable, notamment pour exploiter la détente du gaz propulseur dans le cas d'une valve doseuse.

L'indicateur de dose qui est de préférence réalisé sous la forme d'un compteur de doses, peut être adapté à compter le nombre de doses restant à distribuer ou le nombre de doses déjà distribuées à partir du réservoir 10.

Avantageusement, comme notamment représenté sur la figure 3, l'indicateur a une structure mince comportant l'afficheur 21 et le circuit électronique 25, de sorte qu'il est adaptable à tout dispositif de distribution sans modifier ses dimensions externes. Dans l'exemple des figures 2 et 3, l'afficheur est simplement intégré à l'intérieur du corps 1, au niveau d'une fenêtre pour permettre à l'utilisateur de lire les indications inscrites sur ledit afficheur.

Bien que décrite en référence à des modes de réalisation particuliers, l'invention n'est évidemment pas limitée aux exemples représentés, et un homme du métier peut y apporter toute modification utile sans sortir du cadre de la présente invention telle que définie par les revendications annexées.

## Revendications

1. Dispositif de distribution de produit fluide comportant un corps (1) incorporant un orifice de distribution (5), un réservoir (10) contenant le produit fluide, et un organe de distribution (15), tel qu'une pompe ou une valve doseuse pour distribuer sélectivement le produit contenu dans le réservoir (10), **caractérisé en ce que** le dispositif comporte un indicateur de dose comprenant des moyens d'affichage électroniques (20), lesdits moyens d'affichage (20) comportant un afficheur permanent (21) ne nécessitant aucune énergie pour maintenir l'affichage inchangé et ne nécessitant qu'une très faible énergie pour modifier ledit affichage.

2. Dispositif selon la revendication 1, dans lequel l'afficheur (21) est du type à cristaux liquides (LCD).

3. Dispositif selon la revendication 1 ou 2, dans lequel l'afficheur (21) comporte des cristaux nématiques bistables.

4. Dispositif selon l'une quelconque des revendications précédentes, dans lequel l'énergie nécessaire pour modifier l'affichage est fournie par une pile ou un accumulateur.

5. Dispositif selon l'une quelconque des revendications 1 à 3, dans lequel l'énergie nécessaire pour modifier l'affichage est créé lors de l'actionnement du dispositif.

6. Dispositif selon la revendication 5, dans lequel l'interaction entre deux parties (10, 11 ; 1, 2) du dispositif se déplaçant l'une par rapport à l'autre lors de l'actionnement du dispositif est transformée par un convertisseur électromécanique en une impulsion électrique utilisée pour modifier l'affichage.

7. Dispositif selon la revendication 6, dans lequel ladite interaction est un frottement ou une percussion d'une partie (10, 11) du dispositif sur une autre partie (1, 2) du dispositif lors de l'actionnement.

8. Dispositif selon la revendication 7, dans lequel le réservoir (10) est déplaçable lors de l'actionnement par rapport au corps (1) du dispositif, ledit corps (1) comportant un contacteur (2) coopérant avec ledit réservoir (10), l'interaction entre ledit réservoir (10) et ledit contacteur (2) créant l'impulsion électrique nécessaire pour modifier l'affichage.

9. Dispositif selon la revendication 7, dans lequel un percuteur (11) est déplacé contre un contacteur (2) lors de l'actionnement du dispositif, ledit contacteur (2) étant fixe par rapport audit corps (1), et ledit percuteur (11) coopérant avec un ressort (12).

10. Dispositif selon l'une quelconque des revendications précédentes, dans lequel ledit indicateur de dose indique le nombre de doses de produit distribué ou restant à distribuer du réservoir.

11. Dispositif selon l'une quelconque des revendications précédentes, dans lequel ledit indicateur de dose a une structure mince, de sorte qu'il est adaptable à un dispositif de distribution de produit fluide sans modifier ses dimensions externes.

## Claims

1. A fluid dispenser device comprising a body (1) incorporating a dispenser orifice (5), a reservoir (10) containing the fluid, and a dispenser member (15), such as a metering valve or pump, for selectively dispensing the fluid contained in the reservoir (10), the device being **characterized in that** it further comprises a dose indicator comprising electronic display means (20), said display means (20) including a permanent display member (21) that does not require any energy in order to keep the display unchanged, and that requires only a small amount of energy in order to change said display.

2. A device according to claim 1, in which the display member (21) is of the liquid crystal display (LCD) type.

3. A device according to claim 1 or claim 2, in which the display member (21) includes bistable nematic crystals.

4. A device according to any preceding claim, in which the energy required to change the display is provided by a battery that is optionally rechargeable.

5. A device according to any one of claims 1 to 3, in which the energy required to change the display is created while the device is being actuated.

6. A device according to claim 5, in which the interaction between two portions (10, 11; 1, 2) of the device moving relative to each other while the device is being actuated, is transformed by an electromechanical converter into an electric pulse used to change the display.

7. A device according to claim 6, in which said interaction involves one portion (10, 11) of the device rubbing or striking against another portion (1, 2) of the device during actuation.

8. A device according to claim 7, in which the reservoir (10) is displaceable relative to the body (1) of the device during actuation, said body (1) including a contactor (2) co-operating with said reservoir (10), the interaction between said reservoir (10) and said contactor (2) creating the electric pulse required to change the display.

9. A device according to claim 7, in which a striker pin (11) is displaced against a contactor (2) while the device is being actuated, said contactor (2) being unable to move relative to said body (1), and said striker pin (11) co-operating with a spring (12).

10. A device according to any preceding claim, in which said dose indicator indicates the number of doses of fluid that have been dispensed or that remain to be dispensed from the reservoir.

11. A dispenser according to any preceding claim, in which said dose indicator is thin in structure so that it is adaptable to a fluid dispenser device without having to modify the outside dimensions thereof.

## Patentansprüche

1. Vorrichtung zum Abgeben eines Fluidprodukts, aufweisend einen Körper (1), der eine Abgabeöffnung (5) enthält, einen Vorratsbehälter (10), der das Fluidprodukt aufnimmt, und ein Abgabeorgan (15), wie etwa eine Pumpe oder ein Dosierventil, zum selektiven Abgeben des Produkts, das in dem Vorratsbehälter (10) aufgenommen ist, **dadurch gekennzeichnet, dass** die Vorrichtung eine Dosisanzeigeeinrichtung aufweist, die elektronische Anzeigemittel (20) umfasst, wobei die Anzeigemittel (20) eine dauerhafte Anzeige (21) aufweisen, die keinerlei Energie zur Aufrechterhaltung der ungeänderten Anzeige benötigt, und die zum Modifizieren der Anzeige lediglich eine sehr geringe Energie erfordert.

2. Vorrichtung nach Anspruch 1, wobei die Anzeige (21) eine Flüssigkristallanzeige (LCD) ist.

3. Vorrichtung nach Anspruch 1 oder 2, wobei die Anzeige (21) bistabile nematische Kristalle aufweist.

4. Vorrichtung nach einem der vorangehenden Ansprüche, wobei die zum Modifizieren der Anzeige benötigte Energie durch eine Batterie oder einen Akkumulator geliefert wird.

5. Vorrichtung nach einem der Ansprüche 1 bis 3, wobei die zum Modifizieren der Anzeige erforderliche Energie mit der Betätigung der Vorrichtung erzeugt wird.

6. Vorrichtung nach Anspruch 5, wobei die Wechselwirkung zwischen den beiden Teilen (10, 11; 1, 2) der Vorrichtung, die sich bei Betätigung der Vorrichtung in Bezug aufeinander verlagern, durch einen elektromechanischen Wandler in eine elektrische Impulsfolge umgesetzt wird, die zum Modifizieren der Anzeige genutzt wird.

7. Vorrichtung nach Anspruch 6, wobei die Wechselwirkung im Reiben oder Stoßen von einem Teil (10, 11) der Vorrichtung am bzw, an einen anderen Teil (1, 2) der Vorrichtung bei der Betätigung beruht.

8. Vorrichtung nach Anspruch 7, wobei der Vorratsbehälter (10) bei der Betätigung in Bezug auf den Körper (1) der Vorrichtung verlagerbar ist, wobei der Körper (1) einen Kontaktgeber (2) aufweist, der mit dem vorratsbehälter (10) zusammenwirkt, wobei die Wechselwirkung zwischen dem Vorratsbehälter (10) und dem Kontaktgeber (2) die zum Modifizieren der Anzeige erforderliche elektrische Impulsfolge erzeugt.

9. Vorrichtung nach Anspruch 7, wobei ein Schlagbolzen (11) bei der Betätigung der Vorrichtung gegen einen Kontaktgeber (2) verschoben wird, wobei der Kontaktgeber (2) in Bezug auf den Körper (1) stationär ist, und wobei der Schlagbolzen (11) mit einer Feder (12) zusammenwirkt.

10. Vorrichtung nach einem der vorangehenden Ansprüche, wobei die Dosisanzeige die Anzahl von Produktdosen anzeigt, die abgegeben werden oder im Vorratsbehälter zur Abgabe verbleiben.

11. Vorrichtung nach einem der vorangehenden Ansprüche, wobei die Dosisanzeige eine Struktur kleiner Bauform aufweist, so dass sie auf eine Vorrichtung zur Abgabe von Fluidprodukt anwendbar ist, ohne deren Außenabmessungen zu modifizieren.
